# EUROPEAN PATENT SPECIFICATION

(11) **EP 2 139 382 B1**
(45) Date of publication and mention of the grant of the patent: **19.09.2018**
(21) Application number: 08732645.0
(22) Date of filing: 21.03.2008
(51) Int. Cl.: A61B 5/00, A61M 5/172

(54) **CLOSED LOOP/SEMI-CLOSED LOOP THERAPY MODIFICATION SYSTEM**
THERAPIEÄNDERUNGSSYSTEM MIT GESCHLOSSENEM/HALBGESCHLOSSENEM REGELKREIS
SYSTÈME DE MODIFICATION THÉRAPEUTIQUE EN BOUCLE FERMÉE/BOUCLE SEMI-FERMÉE

(30) Priority: 25.04.2007 US 739927
(43) Date of publication of application: 06.01.2010
(73) Proprietor: Medtronic MiniMed, Inc., Northridge, CA 91325-1219 (US)
(72) Inventor: MASTROTOTARO, John J., Los Angeles, CA 90024 (US); YOON, Richard K., Northridge, CA 91326 (US)
(74) Representative: Goddar, Heinz J.
(86) International application number: PCT/US2008/057807
(87) International publication number: WO 2008/134146

(56) References cited:
- EP-A- 1 759 726
- WO-A-2008/030347
- US-A1- 2003 050 621
- US-A1- 2003 060 753

## Description

### FIELD

This invention relates to infusion systems in closed loop or semi-closed loop applications and more specifically to systems for countering the over/under delivery of medication.

### BACKGROUND

Diabetes mellitus is the most common of endocrine disorders, and is characterized by inadequate insulin action. Diabetes mellitus has two principal variants, known as Type 1 diabetes and Type 2 diabetes. The latter is also referred to as DM/II (diabetes mellitus type 2), adult-onset diabetes, maturity-onset diabetes, or NIDDM (non-insulin dependent diabetes mellitus).

Over the years, body characteristics have been determined by obtaining a sample of bodily fluid. For example, diabetics often test for blood glucose levels. Traditional blood glucose determinations have utilized a finger prick method using a lancet to withdraw a small blood sample. These systems are designed to provide data at discrete points but do not provide continuous data to show variations in the characteristic between testing times. These discrete measurements are capable of informing a patient the state of his blood glucose values at a point in time. Thus, the patient has enough information to administer "correction" amounts of insulin to reduce his current blood glucose reading. However, these discrete readings are not able to provide enough information for any type of automatic or semiautomatic system of administering insulin based on blood glucose values.

Recently, a variety of implantable electrochemical sensors have been developed for detecting and/or quantifying specific agents or compositions in a patient's blood or interstitial fluid. For instance, glucose sensors are being developed for use in obtaining an indication of blood glucose levels in a diabetic patient. These glucose sensors connected (wired or wirelessly) to a blood glucose monitor can provide continuous glucose readings over a period of time, such as 3 to 5 days. Such readings are useful in monitoring and/or adjusting a treatment regimen which typically includes the regular administration of insulin to the patient.

Thus, blood glucose readings improve medical therapies with semi-automated medication infusion pumps of the external type, as generally described in U.S. Pat. Nos. 4,562,751; 4,678,408; and 4,685,903; or automated implantable medication infusion pumps, as generally described in U.S. Pat. No. 4,573,994 are already known. Typical thin film sensors are described in commonly assigned U.S. Pat. Nos. 5,390,671; 5,391,250; 5,482,473; and 5,586,553. See also U.S. Pat. No. 5,299,571. In addition, characteristic glucose monitors used to provide continuous glucose data are described in commonly assigned U.S. patent application Ser. No. 11/322,568 entitled "Telemetered Characteristic Monitor System and Method of Using the Same" filed on Dec. 30, 2005. In addition, infusion pumps receiving sensor data are described in commonly assigned U.S. patent application Ser. No. 10/867,529 entitled "System for Providing Blood Glucose Measurements to an Infusion Device" filed on Oct. 14, 2004.

As sensor technology improves, there is greater desire to use the sensor values to control the infusion of drugs and medicine, such as insulin in a closed loop or semi-closed loop system. Specifically, a closed loop system for diabetes entails a glucose sensor and an insulin infusion pump attached to a patient, wherein the delivery of insulin is automatically administered by a controller of the infusion pump based on the sensor's glucose value readings. A semi-closed system typically includes a patient intervention step, wherein the amount of insulin to be infused as calculated by the controller of the infusion pump requires patient acceptance before delivery.

However, given the ramifications of over-delivery and/or under-delivery of medication, there has yet to be developed a working closed loop/semi-closed loop system that establishes safeguards for countering the over-delivery and/or under-delivery. Therefore, what is needed is a system that places boundaries on a maximum increase or decrease of medication infused into the patient.

US 2003/0050621 A1 discloses a system and a process for providing safety limits on the delivery of an infusion formulation by an infusion pump system in response to a sensed glucose level. A closed loop algorithm continuously checks for changes in glucose level, and if a change occurs, the algorithm determines whether the amount of insulin formulation required based on the change in glucose levels is within normal basal limits. The normal basal limits are pre-programmed into the algorithm. If the measured basal rate is not within the basal limits, the user of the system is notified.

Further systems and methods for the delivery and monitoring of insulin including a continuous measurement of the glucose levels are known from US 2003/0060753 A1 and EP 1 759 726 A2.

### SUMMARY

The present invention provides an infusion system for providing therapy modification in accordance with claim 1.

According to the invention, a closed loop/semi-closed loop infusion system for providing intelligent therapy modification is described. The system of the present invention is configured to obtain a measured blood glucose value and to compare the measured blood glucose value to a target blood glucose value. The system adjusts a therapy delivery parameter if the measured blood glucose value is not consistent with the target blood glucose value, wherein the adjusted therapy delivery parameter is limited to be within a boundary. Thereafter, therapy is delivered at the adjusted therapy delivery parameter.

The system further comprises performing a safety action if the adjusted therapy delivery parameter exceeds the boundary. The therapy delivery parameter is a basal rate.

In the invention, the safety action comprises reviewing a blood glucose history and performing a standard deviation analysis on the blood glucose history to confirm whether the measured blood glucose value is relatively consistent with the blood glucose history. In one embodiment, the standard deviation analysis is performed if the adjusted therapy delivery parameter will increase the delivery of insulin.

In an embodiment of the invention, the safety action comprises notifying a patient of the adjusted therapy delivery parameter. In one embodiment, the patient is notified of the adjusted therapy delivery parameter with an alarm. In another embodiment, the method further comprises prompting the patient to accept the adjusted therapy delivery parameter.

In a further embodiment, the safety action comprises prompting a patient to perform a blood glucose strip meter reading to confirm whether the measured blood glucose value is accurate.

Preferably, a method comprises logging the adjusted therapy delivery parameter. Preferably, the measured blood glucose value is obtained by a glucose sensor system.

In one embodiment, the boundary comprises 25% above or below a preset basal rate. In another embodiment, the boundary comprises a maximum allowed increase or decrease of a preset basal rate. In an alternative embodiment, an absolute value of the maximum allowed increase does not equal an absolute value of the maximum allowed decrease.

According to another embodiment of the invention, the system comprises a glucose sensor system configured to obtain a measured blood glucose value and a controller operationally connected with the glucose sensor system. The controller is configured to compare the measured blood glucose value to a target blood glucose value, adjust a therapy delivery parameter if the measured blood glucose value is not consistent with the target blood glucose value, wherein the adjusted therapy delivery parameter is limited to be within a boundary, compare the adjusted therapy delivery parameter to the boundary, and perform a safety action if the adjusted therapy delivery parameter exceeds the boundary.

The system further comprises a delivery system operationally connected with the controller and configured to deliver therapy at the adjusted therapy delivery parameter. The therapy delivery parameter is a basal rate.

In the invention, the controller performs the safety action by reviewing a blood glucose history and performing a standard deviation analysis on the blood glucose history to confirm whether the measured blood glucose value is relatively consistent with the blood glucose history. In one embodiment, the controller performs the standard deviation analysis if the adjusted therapy delivery parameter will increase the delivery of insulin.

In another embodiment, the controller performs the safety action by notifying a patient of the adjusted therapy delivery parameter. In one embodiment, the system further comprises an alarm for notifying the patient of the adjusted therapy delivery parameter. In another embodiment, the controller prompts the patient to accept the adjusted therapy delivery parameter.

In a further embodiment, the controller performs the safety action by prompting a patient to perform a blood glucose strip meter reading to confirm whether the measured blood glucose value is accurate.

Preferably, the controller logs the adjusted therapy delivery parameter.

In one embodiment, the boundary comprises 25% above or below a preset basal rate. In another embodiment, the boundary comprises a maximum allowed increase or decrease of a preset basal rate. In an alternative embodiment, an absolute value of the maximum allowed increase does not equal an absolute value of the maximum allowed decrease.

According to the invention, the system comprises means for obtaining a measured blood glucose value and means for comparing the measured blood glucose value to a target blood glucose value. The system also comprises means for adjusting a basal rate if the measured blood glucose value is not consistent with the target blood glucose value, wherein the adjusted basal rate is limited to be within a boundary. The system further comprises means for delivering insulin at the adjusted basal rate.

Other features and advantages of the invention will become apparent from the following detailed description, taken in conjunction with the accompanying drawings which illustrate, by way of example, various features of embodiments of the invention.

### BRIEF DESCRIPTION OF THE DRAWINGS

A detailed description of embodiments of the invention will be made with reference to the accompanying drawings, wherein like numerals designate corresponding parts in the several figures.
FIG. 1 is a block diagram of a closed loop glucose control system in accordance with an embodiment of the present invention.
FIG. 2 is a front view of a closed loop system located on a body.
FIG. 3(a) is a perspective view of a glucose sensor system for use in an embodiment of the present invention.
FIG. 3(b) is a side cross-sectional view of the glucose sensor system of FIG. 3(a).
FIG. 3(c) is a perspective view of a sensor set of the glucose sensor system of FIG. 3 (a) for use in an embodiment of the present invention.
FIG. 3(d) is a side cross-sectional view of the sensor set of FIG. 3(c).
FIG. 4 is a cross sectional view of a sensing end of the sensor of FIG 3(d).
FIG. 5 is a perspective view illustrating an example of a subcutaneous sensor insertion set and telemetered characteristic monitor transmitter device when mated together in relation to a characteristic monitor system.
FIG. 6 is a top view of the subcutaneous sensor insertion set and telemetered characteristic monitor transmitter device when separated.
FIG. 7 is a top view of an infusion device with a reservoir door in the open position, for use in an embodiment of the present invention.
FIG. 8 is a side view of an infusion set with the insertion needle pulled out, for use in an embodiment of the present invention.
FIGS. 9(a) and 9(b) are block diagrams of a closed loop glucose control system in accordance with an embodiment of the present invention.
FIG. 10 is a block diagram of auto blood withdrawal and return.
FIG. 11 is an example of a basal rate profile broken up into three-hour intervals in accordance with an embodiment of the present invention.
FIG. 12 is a flowchart illustrating a method for therapy modification in a closed loop/semi-closed loop infusion system in accordance with an embodiment of the present invention.

### DETAILED DESCRIPTION

As shown in the drawings for purposes of illustration, the invention is embodied in a closed loop/semi-closed infusion system for regulating the rate of fluid infusion into a body of a user based on feedback from an analyte concentration measurement taken from the body. The invention is embodied in a control system for regulating the rate of insulin infusion into the body of a user based on a glucose concentration measurement taken from the body.

The system is preferably designed to model a pancreatic beta cell (β-cell). In other words, the system controls an infusion device to release insulin into a body of a user in a similar concentration profile as would be created by fully functioning human β-cells when responding to changes in blood glucose concentrations in the body.

Thus, the system simulates the body's natural insulin response to blood glucose levels and not only makes efficient use of insulin, but also accounts for other bodily functions as well since insulin has both metabolic and mitogenic effects. However, algorithms used must model the β-cells closely. Because the algorithms are designed to minimize glucose excursions in the body, without regard for how much insulin is delivered, the algorithms may cause excessive weight gain, hypertension, and atherosclerosis.

In preferred embodiments of the present invention, the system is intended to emulate the *in vivo* insulin secretion pattern and to adjust this pattern consistent with the *in vivo* β-cell adaptation experienced by normal healthy individuals. The *in vivo* β-cell response in subjects with normal glucose tolerance (NGT), with widely varying insulin sensitivity (S_{I}), is the optimal insulin response for the maintenance of glucose homeostasis.

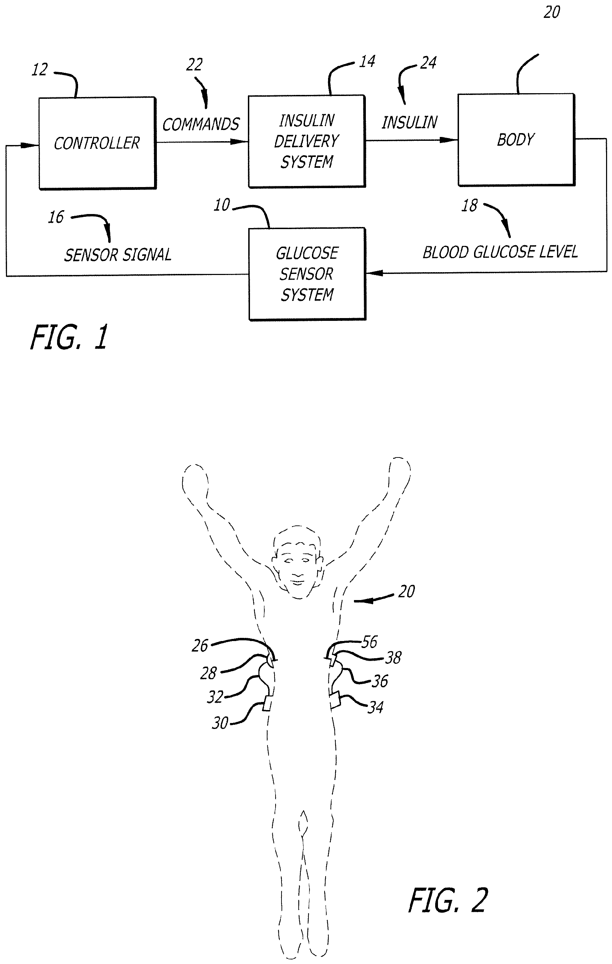

The present invention includes a glucose sensor system 10, a controller 12 and an insulin delivery system 14, as shown in FIG. 1. The glucose sensor system 10 generates a sensor signal 16 representative of blood glucose levels 18 in the body 20, and provides the sensor signal 16 to the controller 12. The controller 12 receives the sensor signal 16 and generates commands 22 that are communicated to the insulin delivery system 14. The insulin delivery system 14 receives the commands 22 and infuses insulin 24 into the body 20 in response to the commands 22. In an alternative semi-closed loop embodiment, the commands 22 would have to be confirmed by the user before the insulin delivery system 14 would infuse insulin.

Generally, the glucose sensor system 10 includes a glucose sensor, sensor electrical components to provide power to the sensor and generate the sensor signal 16, a sensor communication system to carry the sensor signal 16 to the controller 12, and a sensor system housing for the electrical components and the sensor communication system.

Typically, the controller 12 includes controller electrical components and software to generate commands for the insulin delivery system 14 based on the sensor signal 16, and a controller communication system to receive the sensor signal 16 and carry commands to the insulin delivery system 14.

The insulin delivery system 14 preferably includes an infusion device and an infusion tube to infuse insulin 24 into the body 20. For example, the infusion device includes infusion electrical components to activate an infusion motor according to the commands 22, an infusion communication system to receive the commands 22 from the controller 12, and an infusion device housing to hold the infusion device.

In preferred embodiments, the controller 12 is housed in the infusion device housing and the infusion communication system is an electrical trace or a wire that carries the commands 22 from the controller 12 to the infusion device. In alternative embodiments, the controller 12 is housed in the sensor system housing and the sensor communication system is an electrical trace or a wire that carries the sensor signal 16 from the sensor electrical components to the controller electrical components. In other alternative embodiments, the controller 12 has its own housing or is included in a supplemental device. In another alternative embodiment, the controller is located with the infusion device and the sensor system all within one housing. In further alternative embodiments, the sensor, controller, and/or infusion communication systems may utilize a cable, a wire, fiber optic lines, RF, IR, or ultrasonic transmitters and receivers, or the like instead of the electrical traces.

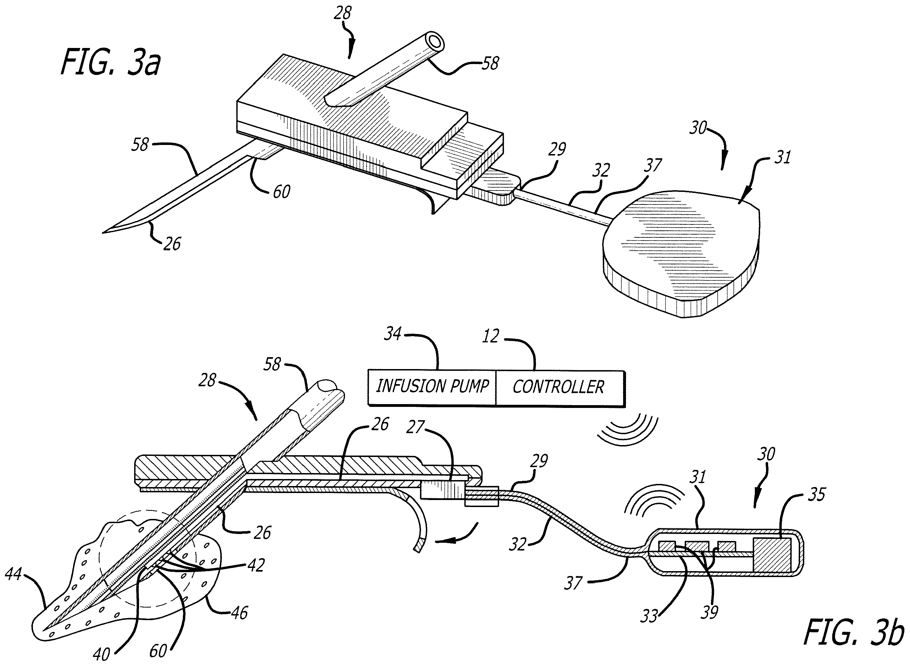

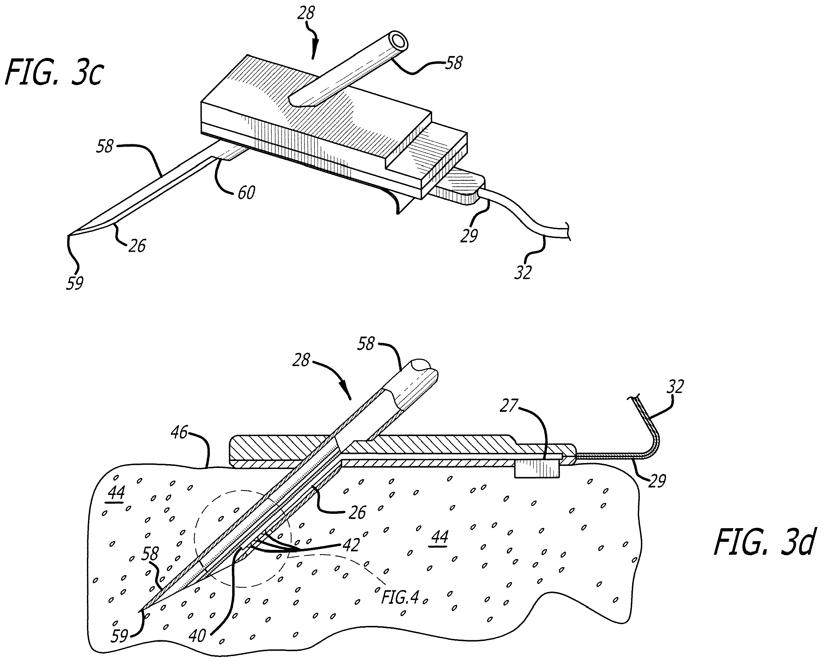

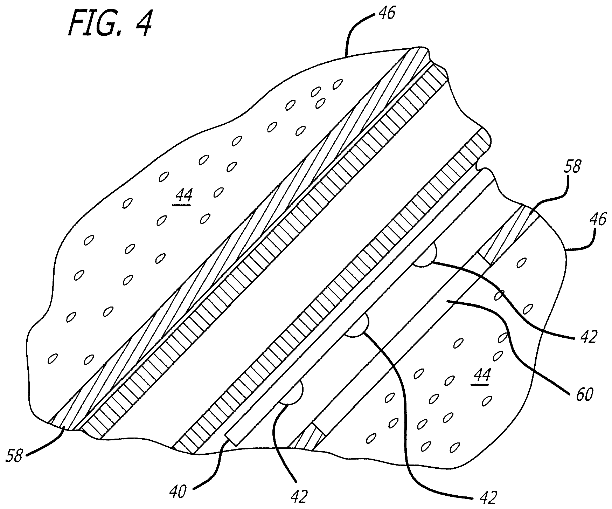

FIG. 2 is a front view of a closed loop system located on a body in accordance with an embodiment of the present invention. Referring to FIG. 2, the closed loop system includes a sensor 26, a sensor set 28, a telemetered characteristic monitor transmitter 30, a sensor cable 32, an infusion pump 34, an infusion tube 36, and an infusion set 38, all worn on the body 20 of a user. Referring to FIGS. 3(a) and 3(b), the telemetered characteristic monitor transmitter 30 includes a transmitter housing 31 that supports a printed circuit board 33, batteries 35, antenna (not shown), and a sensor cable connector (not shown). Referring to FIGS. 3(b), 3(d) and 4, a sensing end 40 of the sensor 26 has exposed electrodes 42 and is inserted through skin 46 into a subcutaneous tissue 44 of a user's body 20. The electrodes 42 are in contact with interstitial fluid (ISF) that is present throughout the subcutaneous tissue 44. Referring to FIGS. 3(b) and 3(d), the sensor 26 is held in place by the sensor set 28, which is adhesively secured to the user's skin 46. The sensor set 28 provides for a connector end 27 of the sensor 26 to connect to a first end 29 of the sensor cable 32. A second end 37 of the sensor cable 32 connects to the transmitter housing 31. The batteries 35 included in the transmitter housing 31 provide power for the sensor 26 and electrical components 39 on the printed circuit board 33. The electrical components 39 sample the sensor signal 16 and store digital sensor values (Dsig) in a memory and then periodically transmit the digital sensor values Dsig from the memory to the controller 12, which is included in the infusion device.

As shown in FIGS. 3(a)-3(d), the telemetered characteristic monitor transmitter 30 is coupled to a sensor set 28 by a sensor cable 32. In alternative examples, the cable 32 may be omitted, and the telemetered characteristic monitor transmitter 30 may include an appropriate connector for direct connection to the connector portion 27 of the sensor set 28 or the sensor set 28 may be modified to have the connector portion 27 positioned at a different location.

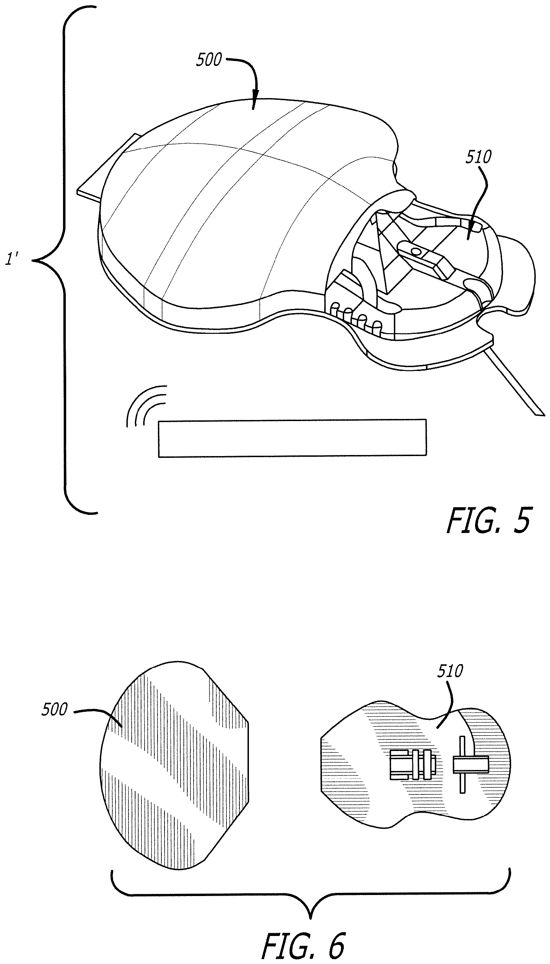

For example, FIGS. 5 and 6 show a possible alternative example where characteristic monitor transmitter 500 and the sensor set 510 can be modified to allow a side-by side direct connection between the characteristic monitor transmitter 500 and the sensor set 510 such that the characteristic monitor transmitter 500 is detachable from the sensor set 510, as seen in FIG. 6. Another possible example (not shown) can modify the top of the sensor set 510 to facilitate placement of the telemetered characteristic monitor transmitter 500 over the sensor set 510.

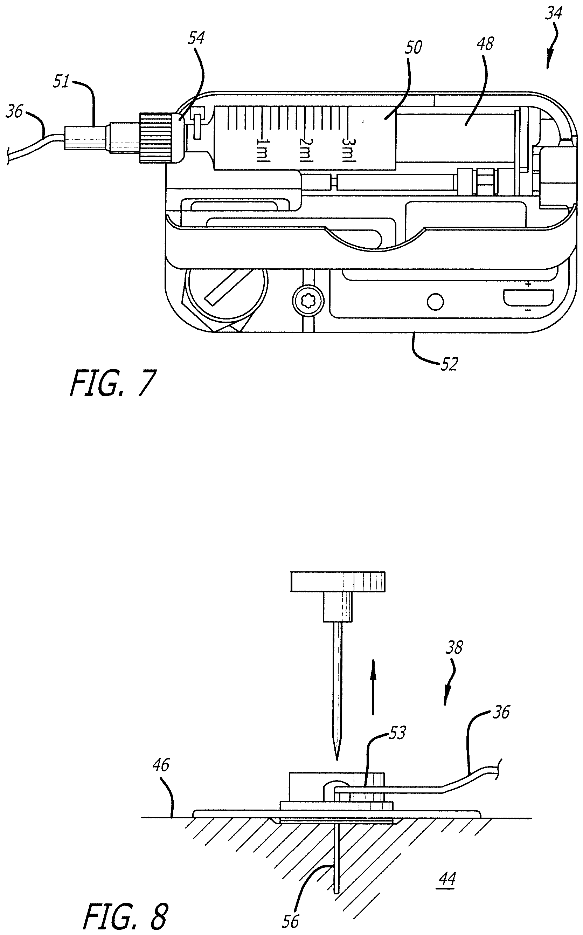

FIG. 7 is a top view of an infusion device with a reservoir door in the open position, for use in an embodiment of the present invention. Referring to FIGS. 1 and 7, the controller 12 processes the digital sensor values Dsig and generates commands 22 for the infusion pump 34. Preferably, the infusion device 34 responds to the commands 22 and actuates a plunger 48 that forces insulin 24 out of a reservoir 50 located inside the infusion device 34. In particular embodiments, a connector tip 54 of the reservoir 50 extends through the infusion device housing 52 and a first end 51 of the infusion tube 36 is attached to the connector tip 54. A second end 53 of the infusion tube 36 connects to the infusion set 38. Referring to FIGS. 2, 7 and 8, insulin 24 is forced through the infusion tube 36 into the infusion set 38 and into the body 20. The infusion set 38 is adhesively attached to the user's skin 46. As part of the infusion set 38, a cannula 56 extends through the skin 46 and terminates in the subcutaneous tissue 44 completing fluid communication between the reservoir 50 and the subcutaneous tissue 44 of the user's body 20.

In alternative embodiments, the closed-loop/semi-closed loop system can be a part of a hospital-based glucose management system. Given that insulin therapy during intensive care has been shown to dramatically improve wound healing, reduce blood stream infections, renal failure, and polyneuropathy mortality, irrespective of whether subjects previously had diabetes, the present invention can be used in this hospital setting to control the blood glucose level of a patient in intensive care.

In these alternative embodiments, since an IV hookup is typically implanted into a patient's arm while the patient is in an intensive care setting (e.g. ICU), a closed loop glucose control can be established which piggy-backs off the existing IV connection. Thus, in a hospital based system, intravenous (IV) catheters which are directly connected to a patient vascular system for purposes of quickly delivering IV fluids, can also be used to facilitate blood sampling and direct infusion of substances (e.g. insulin, anticoagulants) into the intra-vascular space. Moreover, glucose sensors may be inserted through the IV line to give real-time glucose levels from the blood stream.

Therefore, depending on the type of hospital based system, the alternative embodiments would not necessarily need the described system components, such as the sensor 26, the sensor set 28, the telemetered characteristic monitor transmitter 30, the sensor cable 32, the infusion tube 36, and the infusion set 38 as described in the preferred embodiments. Instead, standard blood glucose meters or vascular glucose sensors as described in patent application entitled "Multi-lumen Catheter," filed December 30, 2002, Serial Number 10/331,949, can be used to provide the blood glucose values to the infusion pump control and the existing IV connection can be used to administer the insulin to the patient.

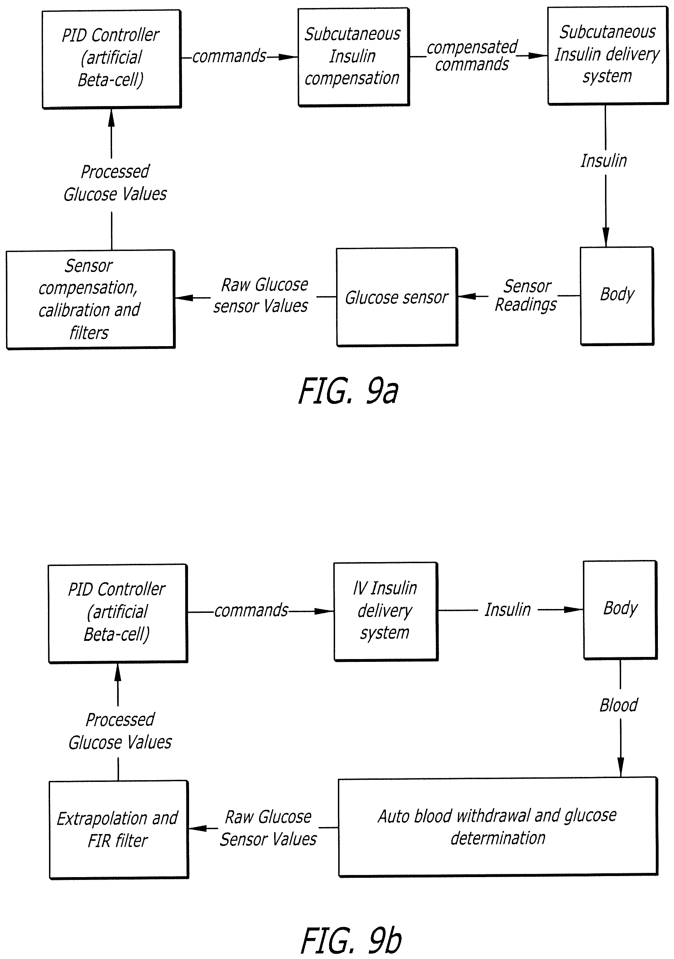

It is important to appreciate that numerous combinations of devices in the hospital-based system can be used with the closed loop controller of the present invention. FIGS. 9(a) and 9(b) illustrate examples of such combinations. Specifically, FIG. 9(a) illustrates a subcutaneous sensor system. FIG. 9(b) illustrates an auto blood glucose/intravenous insulin infusion system that can automatically withdraw and analyze blood for glucose concentration at fixed intervals (preferably 5 - 20 minutes), extrapolate the blood glucose values at a more frequent interval (preferably 1 minute), and use the extrapolated signal for calculating an iv-insulin infusion according to the controller described below.

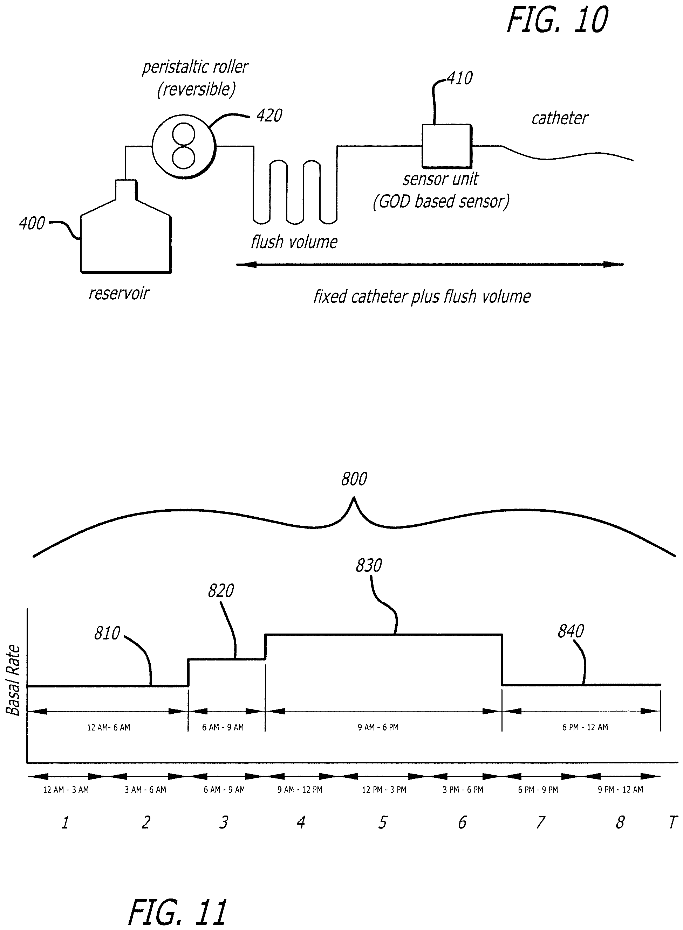

The modified auto blood glucose/intravenous insulin infusion system would eliminate the need for subcutaneous sensor compensation and subcutaneous insulin compensation which would be required with a subcutaneous sensor system (as described below when discussing the delay problems inherent in a subcutaneous sensor system). The automatic withdrawal of blood, and subsequent glucose determination can be accomplished with existing technology (e.g. VIA or Biostator like blood glucose analyzer) or by the system described in FIG. 10.

FIG. 10 is a block diagram of auto blood withdrawal and return. The system in FIG. 10 uses a peristaltic pump 420 to withdraw blood across an amperometric sensor 410 (the same technology as used in sensor 26) and then return the blood with added flush (0.5 to 1.0 ml) from the reservoir 400. The flush may comprise of any makeup of saline, heparin, glucose solution and/or the like.

If the blood samples are obtained at intervals longer than 1 minute but less than 20 minutes, the blood glucose determinations can be extrapolated on a minute-to-minute basis with extrapolation based on the present (n) and previous values (n-1) to work with the logic of the controller as described in detail below. For blood samples obtained at intervals greater than 20 minutes, a zero-order-hold would be used for the extrapolation. Based on these blood glucose values, the infusion device can administer insulin based on the closed loop controller described in greater detail below.

In other modifications to the system, a manual blood glucose/intravenous insulin infusion system can be used where frequent manual entries of blood glucose values from a standard blood glucose meter (e.g., YSI, Beckman, etc) are extrapolated at more frequent intervals (preferably 1 min) to create a surrogate signal for calculating IV-insulin infusion. Alternatively, a sensor blood glucose/intravenous insulin infusion system can use a continuous glucose sensor (e.g., vascular, subcutaneous, etc.) for frequent blood glucose determination. Moreover, the insulin infusion can be administered subcutaneously rather than intravenously in any one of the previous examples according to the controller described below.

In still further alternative examples, the system components may be combined in a smaller or greater number of devices and/or the functions of each device may be allocated differently to suit the needs of the user.

Once the hardware for a closed loop/semi-closed loop system is configured, such as in the preferred embodiments and examples described above, the affects of the hardware on a human body are determined by the controller. In preferred embodiments, the controller 12 is designed to model a pancreatic beta cell (β-cell).

In other words, the controller 12 commands the infusion device 34 to release insulin 24 into the body 20 at a rate that causes the insulin concentration in the blood to follow a similar concentration profile as would be caused by fully functioning human β-cells responding to blood glucose concentrations in the body 20. Thus, the controller 12 is intended to emulate the *in vivo* insulin secretion pattern and to adjust this pattern to be consistent with *in vivo* β-cell adaptation.

The *in vivo* β-cell response in subjects with normal glucose tolerance (NGT), with widely varying insulin sensitivity (S_{I}), is the optimal insulin response for the maintenance of glucose homeostasis. The biphasic insulin response of a β-cell can be modeled using components of a proportional, plus integral, plus derivative (PID) controller along with various filters. Description of a PID controller to emulate β-cells can be found in commonly assigned U.S. Patent No. 6,558,351.

In alternative embodiments, the controller may simply be the controller in an infusion pump that calculates the amount of insulin to be infused based upon the insulin sensitivity/carbohydrate ratio of the individual, the target blood glucose level, amount of carbohydrates to be ingested and the current blood glucose level supplied by the sensor. An example of such a controller is described in commonly assigned U.S. Patent No. 6,554,798 entitled "External Infusion Device with Remote Programming, Bolus Estimator and/or Vibration Alarm Capabilities,". In additional embodiments, the controller may simply be a controller of an infusion pump which takes an additional input of blood glucose values from a sensor or meter, where the controller solely relies on the current glucose value (or predicted glucose value) without relying on additional factors such as insulin sensitivity or carbohydrate ratio or carbohydrates ingested. For example, the system may be used only for overnight closed-loop applications, where there is no expectation of any carbohydrates ingested. Instead, the key focus may simply be to prevent hypoglycemic excursions during sleeping times because the immediate risks of hypoglycemia are much greater than hyperglycemia. Hypoglycemia can cause a person to pass out in 15 or 30 minutes while it takes hours for the severe effects of hyperglycemia to become evident and cause problems. In such an application, the controller in the infusion pump could simply lower the basal rate or shut off the basal rate completely to prevent the blood glucose levels from falling to dangerous levels. On the other hand, the controller can also correct for hyperglycemic excursions, by increasing the basal rate simply using only insulin sensitivity factor and current blood glucose value in determining how much additional insulin to deliver.

Yet in further embodiments, the system may be a combination closed loop/open loop system. For example, a controller of an infusion pump can be programmed to go into open loop conditions during meal times (i.e. injections of meal boluses) or correction boluses, where the user will control the amount of insulin given in a bolus. However, the controller will place the system back to a default closed-loop/semi-closed system when the insulin on board from a meal or correction bolus is de minimis (for example, 2 hours).

Regardless of the controller used with the present system, closed loop/semi-closed loop algorithms for insulin delivery rely on a continuous or periodic glucose sensor to drive a control algorithm that determines the optimal insulin dose to administer through a pump delivery mechanism. Therefore sensor reliability and fault detection and handling are crucial to the dependability and safety of such an application.

It is therefore desirable to have an assessment mechanism that can evaluate the sensor signal fidelity and initiate the appropriate action following detection of a sensor failure. In the event a fault is detected, a request for sensor replacements should be initiated and a temporary suspension of insulin delivery or control should switch to a fixed mode of operation with set basal patterns.

One method of identifying whether the sensor values are reliable involves the measure of other signals by the sensor that may provide information about the state of the sensor (such as voltage readings, impedance, etc). Another method to assure an accurate sensor reading is to use a dual or 3-up sensing system located in a single sensor site so that the sensors are used to check one another. Here, the system continues in a closed-loop mode as long as the sensors are in agreement with each other. Moreover, the likelihood of each sensor failing in the same way, or at the same time, is small.

A further method for identifying whether the sensor values are reliable relates to the use of sensor redundancy, wherein the sensing method and/or sensor location of redundant sensors are different from one another. For example, in one example, two subcutaneous sensors located at different sites would assure that the potential for common effects due to sensor location or interferences is negligible.

However, alternative sites may generate different physiological delays that could result from skin temperature or pressure variance at the measuring site. For example, when additional pressure is applied to one of the sites due to sleep posture, the readings may vary. Moreover, two identical sensors that should exhibit the same readings can exhibit varying time lags, sensitivities and offsets leading to confusing signals.

Thus, in further examples, sensors using different technology are placed in different body fluids, e.g. one sensor in subcutaneous tissue and one in blood. Therefore, although the previous description described various types of electro-enzymatic sensors, the system will use other types of sensors, such as chemical based, optical based or the like.

For example, other types of sensors are described in the following references: U.S. Provisional Application Serial No. 60/007,515 to Van Antwerp et al. and entitled "Minimally Invasive Chemically Amplified Optical Glucose Sensor"; U.S. Patent No. 6,011,984 issued January 4, 2000 to Van Antwerp et al. and entitled "Detection of Biological Molecules Using Chemical Amplification"; and U.S. Patent No. 6,766,183 issued July 20, 2004 to Walsh et al. and entitled "Long Wave Flourophore Sensor Compounds and Other Fluorescent Sensor Compounds in Polymers". Other compounds using Donor Acceptor fluorescent techniques may be used, such as disclosed in U.S. Patent No. 5,628,310 issued May 13, 1997 to Rao et al. and entitled " Method and Apparatus to Perform Trans-cutaeous Analyte Monitoring"; U.S. Patent No. 5,342,789 issued August 30, 1994 to Chick et al. and entitled "Method and Device for Detecting and Quantifying Glucose in body Fluids"; and U.S. Patent No. 5,246,867 issued September 21, 1993 to Lakowicz et al. and entitled "Determination and Quantification of Saccharides by Luminescent Lifetimes and Energy Transfer". Hence, use of two different types of sensors at two different locations, may assure failsafe performance of the system that relies heavily on accurate sensor readings.

The insulin delivery system 14 together with the controller 12 mimics the delivery of a normal pancreas. To do so, the insulin delivery system 14 delivers steady amounts of insulin, known as a basal rate, throughout a day. The basal rate delivers the amount of insulin needed in a fasting state to maintain target glucose levels. The basal rate insulin is intended to account for the baseline insulin needs of the body, and makes up approximately fifty percent of the body's total daily insulin requirements.

Thus, similar to the pancreas, the insulin delivery system 14 delivers basal rate insulin continuously over the twenty-four hours in the day. The insulin delivery system 14 can be set to automatically provide one or more different rates during different time intervals of the day. These different basal rates at various time intervals during the day usually depend on a patient's lifestyle and insulin requirements. For example, many insulin delivery system users require a lower basal rate overnight while sleeping and a higher basal rate during the day, or users may want to lower the basal rate during the time of the day when they regularly exercise.

FIG. 11 is an example of a basal rate profile broken up into three-hour intervals in accordance with an embodiment of the present invention. Referring to FIG. 11, the basal pattern 800 can have various basal rates (810, 820, 830, 840) throughout the day, and the basal rates do not necessarily change at each interval. Moreover, adjustments to the specific basal rates can be made for each time interval. Notably, these intervals can be started at any time to match the user's schedule and intervals can be greater or less than three-hours in length. A single basal rate interval can be as short as a minimum basal rate interval capable of being programmed by an insulin delivery system or have a maximum of 24 hours.

A patient's target blood glucose level (Target) is the amount of blood glucose (BG) that the patient wishes to achieve and maintain. Typically, a target blood glucose value is between 70-120 mg/dL for preprandial BG and 100-150 mg/dL for postprandial BG. Thus, the patient's basal pattern is set to achieve and maintain the Target during insulin therapy.

According to an embodiment of the invention, an algorithm may provide intelligent therapy modifications for various pump therapy parameters to help patients more easily achieve and maintain the target blood glucose level. The algorithm automatically adjusts insulin delivery parameters based on the difference between a glycemic target and a measured glucose value.

In the preferred embodiments, the algorithm is incorporated in the controller 12 that is able to receive signals from the glucose sensor system 10. In the preferred embodiments, the algorithm is stored in the controller's firmware, but can be stored in a separate software routine in the controller's memory. In addition, the insulin delivery system 14 is able to run the algorithm to perform the necessary steps to provide intelligent therapy modifications for various pump therapy parameters.

Alternatively, the algorithm can be run on a separate device such as a PDA, smart phone, computer, or the like. In further alternative embodiments, the algorithm can be run on the glucose sensor system 10 or combination glucose sensor system/infusion delivery system or peripheral controller.

In preferred embodiments, the intelligent therapy modifications are displayed on the insulin delivery system 14, whether the modifications themselves were calculated by the controller 12 or sent from another device either by cable or wireless means. However, in alternative embodiments, the therapy recommendations can also be given on any associated device such as a glucose sensor system display, a handheld PDA, a smart phone, a computer, etc.

Considering a patient's varying body characteristics throughout the day, the patient's insulin requirements may diverge from the patient's preset basal pattern if blood glucose levels are not as expected. If so, the insulin delivery system 14 may utilize the control algorithm to determine an optimal amount of insulin to deliver to the patient. Accordingly, the controller 12 can command the insulin delivery system 14 to automatically adjust the basal rate for a given time interval according to the optimal amount determined. Hence, better therapy is provided because the patient's current insulin requirements are addressed.

However, when basal rates are allowed to be automatically changed in a closed loop and/or semi-closed loop system, the insulin delivery system 14 is susceptible to delivering too much or too little insulin to the patient if any devices involved in derivation of the adjusted basal rate fail. For example, if the sensors of the glucose sensor system 10 become faulty, then an inaccurate blood glucose level is detected. Consequently, the controller 12 may command the insulin delivery system 14 to over-deliver or under-deliver insulin to the patient. Thus, safeguards are preferably implemented to ensure against the over-delivery or under-delivery of insulin. Besides the use of multiple sensors, another (or independently used) possible safeguard may be the use of a model predictive supervisory control. A separate algorithm can be used to confirm that blood glucose values are acting as expected based on the delivery of insulin. When the actual values deviate too much from the predictive models, alarms or other corrective actions can be used. Examples of model predictive supervisory algorithms can be seen in commonly assigned U.S. Patent Application No. 11/700,666 entitled "Model Predictive Method and System for Controlling and Supervising Insulin Infusion" filed January 31, 2007. In addition, to the above mentioned safeguards, the present invention provides for an additional (or independently used) safety mechanism to avoid over-delivery or under-delivery of insulin.

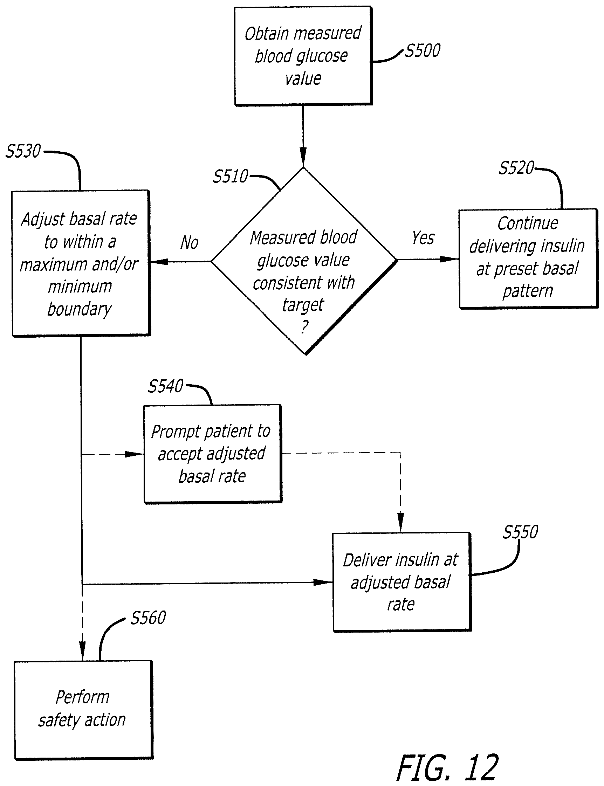

FIG. 12 illustrates a method for providing therapy modification in a closed loop/semi-closed loop infusion system, wherein the over-delivery or under-delivery of insulin is prevented in accordance with the preferred embodiments of the present invention. Referring to FIG. 12, at the end of a particular basal pattern time interval, a patient's measured blood glucose level is obtained (S500) using the glucose sensor system 10 of FIG. 1, for example.

Upon obtaining the measured blood glucose level, the controller 12 determines whether the Target is successfully achieved and maintained (S510). If so, the controller commands the insulin delivery system to continue delivering insulin to the patient according to the patient's preset personal basal pattern (S520). However, if the Target is not achieved or maintained, then the controller 12 will attempt to adjust the basal rate to a temporary adjusted basal rate (S530). Depending on whether the blood glucose is higher or lower than the targeted blood glucose level, more or less insulin will be delivered compared to the existing patient's preset basal rate set in his/her basal pattern .

However, in administering the insulin at the adjusted basal rate, the controller 12 preferably limits the adjusted basal rate to a maximum and/or minimum boundary on the adjusted basal rate (S530). The maximum and/or minimum boundary on the adjusted basal rate is set based on the preset basal rate.

For example, a predefined boundary may be set at 25% above or below the preset basal rate for any given time interval. In the preferred embodiment, 25% is chosen so that the amount of insulin makes only small changes to the blood glucose value over a longer period of time. However, this boundary can be defined at any percentage or value above or below a preset basal rate. Therefore, if the adjusted basal rate is within 25% above or below the preset basal rate, the controller 12 will command the insulin delivery system 14 to deliver insulin at the adjusted basal rate (S550). In a semi-closed system, the insulin delivery system will prompt the patient to accept the adjusted basal rate prior to the delivery of insulin (S540).

Alternatively, if the absolute value of the difference between the adjusted basal rate value and the preset basal rate value is greater than a predefined threshold (i.e. exceeds the maximum/minimum boundary on the preset basal rate), the controller 12 will only adjust the delivery rate up to the boundary value, and cap the maximum or minimum amount of adjustment allowed compared to the patient's preset basal pattern. If the preset basal pattern has programmed an increase or decrease at the next time interval (i.e. the time when the basal rate is preprogrammed to change), the maximum adjustment will move with the change in the preset basal pattern while maintaining the threshold difference between the preset basal rate and the adjusted basal rate.

Over time, if the basal rate is regularly adjusted and consistently reaches the maximum and/or minimum boundary for any given time interval, this may indicate that the patient's insulin requirements have changed, and therefore the patient's personal basal pattern may need to be altered. Accordingly, based on the maximum and/or minimum boundary value consistently reached during any given time interval, the controller 12 may recommend to the patient a new basal rate the patient can integrate into his/her personal basal pattern.

In alternative embodiments, it is contemplated that systematic errors may occur, and therefore the predefined threshold may be exceeded when adjusting the basal rate (i.e. the amount of insulin to be delivered exceeds the maximum/minimum boundary on the preset basal rate). In such a case, the controller does not automatically allow the insulin delivery system 14 to deliver insulin to the patient at the adjusted basal rate. Consequently, at least one of a number of actions will be performed (S560).

According to the invention the adjusted basal rate value is evaluated for safety. According to the invention, the safety review ensures that the glucose history is not too variable for a therapy modification to be made. A therapy modification should only be made if there is a consistent pattern in blood glucose levels to provide a certain level of confidence in the therapy modification.

According to the invention, the control algorithm determines the variability of the glucose history by using a standard deviation of a cluster of the most recent data. The standard deviation is compared against the difference between an average blood glucose value and the target blood glucose value. If the glucose history is too variable for a therapy modification to be made, i.e. the standard deviation is greater than the difference between the average blood glucose value and the target blood glucose value, no therapy modification is made.

According to the invention, the safety check is only applied for increases in the basal rate because the immediate risks of hypoglycemia are much greater than hyperglycemia. Hypoglycemia can cause a person to pass out in 15 to 30 minutes while it takes hours for the severe effects of hyperglycemia to become evident and cause problems.

In another example, if the absolute value of the difference between the adjusted basal rate value and the preset basal rate value is greater than the predefined threshold, the insulin delivery system 14 will notify the patient that the predefined threshold has been exceeded. This may be accomplished visually by informing the patient of the event through a display screen on the insulin delivery system, or by setting off different alarms via audio or vibration.

During notification, the patient may be prompted to either accept the insulin delivery at the adjusted basal rate or asked to manually input a basal rate within the predefined threshold. Additionally, during notification, the patient may be also be asked to perform a blood glucose strip meter reading to confirm whether the measured blood glucose values read by the glucose sensor system are accurate.

In preferred embodiments, all obtained and measured glucose values and basal rates adjusted during a basal pattern are logged. Accordingly, a patient can refer to the log to manage treatment more accurately. For example, the patient can utilize the log to assess where to modify the patient's personal basal pattern during a certain period of time. Moreover, as certain patterns of automatic increase and decrease of basal rates occur, the controller and the insulin delivery system can utilize the log to provide therapy recommendations to the patient for modifying the patient's personal pattern.

While the description above refers to particular embodiments of the present invention, it will be understood that many modifications may be made without departing from the spirit thereof. Thus, the accompanying claims are intended to cover such modifications as would fall within the true scope and spirit of the present invention.

The presently disclosed embodiments are therefore to be considered in all respects as illustrative and not restrictive, the scope of the invention being indicated by the appended claims, rather than the foregoing description, and all changes which come within the meaning and range of equivalency of the claims are therefore intended to be embraced therein.

## Claims

1. An infusion system for providing therapy modification, the system comprising:
a glucose sensor system (10) configured to obtain a measured blood glucose value;
a controller (12) operationally connected with the glucose sensor system (10);
an insulin delivery system operationally connected with the controller (12);
wherein the controller is configured to compare the measured blood glucose value to a target blood glucose value, and adjust a basal rate if the target blood glucose value is not achieved or maintained, wherein the adjusted basal rate is limited to be within a boundary wherein the boundary includes a maximum basal rate set as a percentage of a preset basal rate above the preset basal rate, and a minimum basal rate;
wherein the controller is further configured to perform a safety action if the adjusted basal rate exceeds the boundary;
wherein the controller is further configured to command the insulin delivery system to deliver therapy at the adjusted basal rate if the adjusted basal rate is within the boundary and, if the adjusted basal rate exceeds the boundary, the safety action ensures that the glucose history is not too variable for a therapy modification to be made and the therapy modification is only made if there is a consistent pattern in the blood glucose history, **characterized in that**
the minimum basal rate is set as a percentage of a preset basal rate below the preset basal rate, and
the variability of the glucose history is determined by using a standard deviation of a cluster of the most recent data and comparing this standard deviation against the difference between an average blood glucose value and the target blood glucose value.

2. The system of claim 1, wherein the controller prompts a patient to accept the adjusted basal rate prior to delivering the therapy.

3. The system of claim 1, wherein the controller performs the standard deviation analysis if the adjusted basal rate will increase the delivery of insulin.

4. The system of claim 1, wherein the controller further performs the safety action by notifying a patient of the adjusted basal rate.

5. The system of claim 4, further comprising an alarm for notifying the patient of the adjusted basal rate.

6. The system of claim 4, wherein the controller further prompts the patient to accept the adjusted basal rate.

7. The system of claim 1, wherein the controller further performs the safety action by prompting a patient to perform a blood glucose strip meter reading to confirm whether the measured blood glucose value is accurate.

8. The system of claim 1, wherein the controller logs the adjusted basal rate.

9. The system of claim 1, wherein the boundary comprises 25% above or below a preset basal rate.

10. The system of claim 1, wherein an absolute value of the maximum allowed increase does not equal an absolute value of the maximum allowed decrease.

## Patentansprüche

1. Infusionssystem zur Bereitstellung einer Therapiemodifikation, wobei das System umfasst:
ein Glukosesensorsystem (10), das konfiguriert ist, um einen gemessenen Blutzuckerwert zu erhalten;
einen Regler (12), der betriebsmäßig mit dem Glukosesensorsystem (10) verbunden ist;
ein Insulinverabreichungssystem, das betriebsmäßig mit dem Controller (12) verbunden ist;
wobei die Steuerung so konfiguriert ist, dass sie den gemessenen Blutzuckerwert mit einem Ziel-Blutzuckerwert vergleicht, und eine Basalrate anpasst, wenn der Ziel-Blutzuckerwert nicht erreicht oder aufrechterhalten wird, wobei die angepasste Basalrate begrenzt ist auf einen Wert innerhalb einer Grenze, wobei die Grenze eine maximale Basalrate, die als Prozentsatz von einer voreingestellten Basalrate über der voreingestellten Basalrate gesetzt ist, und eine minimale Basalrate enthält;
wobei die Steuerung weiterhin so konfiguriert ist, dass sie eine Sicherheitsaktion ausführt, wenn die eingestellte Basalrate die Grenze überschreitet;
wobei die Steuerung weiter konfiguriert ist, um das Insulinverabreichungssystem anzuweisen, eine Therapie mit der eingestellten Basalrate durchführen, wenn die eingestellte Basalrate innerhalb der Grenze liegt und, wenn die eingestellte Basalrate der Grenze überschreitet, die Sicherheitsmaßnahme sicherstellt, dass die Glukosehistorie nicht zu variabel ist, um eine Therapieänderung vorzunehmen und die Therapiemodifikation nur bei gleichbleibender Blutzuckerhistorie erfolgt, **dadurch gekennzeichnet, dass** die minimale Basalrate als Prozentsatz einer voreingestellten Basalrate unter der voreingestellten Basalrate eingestellt ist, und
die Variabilität der Glukosehistorie mit Hilfe einer Standardabweichung eines Cluster der aktuellsten Daten und Vergleich dieser Standardabweichung mit der Differenz zwischen einem durchschnittlichen Blutzuckerwert und dem Ziel-Blutzuckerwert bestimmt wird.

2. System nach Anspruch 1, wobei der Regler einen Patienten auffordert, die eingestellte Basalrate vor der Therapie anzunehmen.

3. System nach Anspruch 1, wobei der Regler die Standardabweichungsanalyse durchführt, wenn die angepasste Basalrate die Insulinzufuhr erhöht.

4. System nach Anspruch 1, wobei die Steuerung weiterhin die Sicherheitsmaßnahme durchführt durch Benachrichtigung eines Patienten über die eingestellte Basalrate.

5. System nach Anspruch 4, das ferner einen Alarm zur Benachrichtigung des Patienten über die angepasste Basalrate umfasst.

6. System nach Anspruch 4, wobei der Regler den Patienten weiterhin auffordert, die angepasste Basalrate anzunehmen.

7. System nach Anspruch 1, wobei der Regler weiterhin die Sicherheitsmaßnahme durchführt durch Auffordern eines Patienten, eine Blutzuckermessung durchzuführen, um zu bestätigen, ob der gemessene Blutzuckerwert genau ist.

8. System von Anspruch 1, wobei der Regler die eingestellte Basalrate protokolliert.

9. System nach Anspruch 1, wobei die Grenze 25 % über oder unter einer voreingestellten Basalrate liegt.

10. System des Anspruchs 1, wobei ein absoluter Wert der maximal zulässigen Erhöhung nicht einem absoluten Wert der maximal zulässigen Abnahme entspricht.

## Revendications

1. Système de perfusion pour réaliser une modification thérapeutique, le système comprenant :
un système de détection de glucose (10) configuré pour obtenir une valeur de glycémie mesurée ;
un dispositif de contrôle (12) relié opérationnellement au système de détection de glucose (10) ;
un système d'administration d'insuline relié opérationnellement au dispositif de contrôle (12) ;
dans lequel le dispositif de contrôle est configuré pour comparer la valeur de glycémie mesurée à une valeur de glycémie cible, et rectifier un taux de base si la valeur de glycémie cible n'est pas atteinte ou maintenue, dans lequel le taux de base rectifié est limité de sorte à se trouver dans une fourchette dans laquelle la fourchette inclut un taux de base maximal établi en tant que pourcentage d'un taux de base préétabli supérieur au taux de base préétabli, et un taux de base minimal ;
dans lequel le dispositif de contrôle est configuré en outre pour réaliser une action de sécurité si le taux de base rectifié dépasse la fourchette ;
dans lequel le dispositif de contrôle est configuré en outre pour donner l'ordre au système d'administration d'insuline d'administrer la thérapie au taux de base rectifié si le taux de base rectifié se trouve dans la fourchette et, si le taux de base rectifié dépasse la fourchette, l'action de sécurité garantit que l'historique glycémique n'est pas suffisamment variable pour qu'une modification thérapeutique soit effectuée et la modification thérapeutique n'est effectuée que s'il existe un schéma cohérent dans l'historique glycémique, **caractérisé en ce que**
le taux de base minimal est établi en tant que pourcentage d'un taux de base préétabli en dessous du taux de base préétabli, et
la variabilité de l'historique glycémique est déterminée en utilisant un écart type d'un groupe des données les plus récentes et en comparant cet écart type à la différence entre une valeur de glycémie moyenne et la valeur de glycémie cible.

2. Système selon la revendication 1, dans lequel le dispositif de contrôle demande à un patient d'accepter le taux de base rectifié avant administration de la thérapie.

3. Système selon la revendication 1, dans lequel le dispositif de contrôle réalise l'analyse de l'écart type si le taux de base rectifié augmente l'administration d'insuline.

4. Système selon la revendication 1, dans lequel le dispositif de contrôle réalise en outre l'action de sécurité en indiquant à un patient le taux de base rectifié.

5. Système selon la revendication 4, comprenant en outre une alarme pour indiquer au patient le taux de base rectifié.

6. Système selon la revendication 4, dans lequel le dispositif de contrôle demande en outre au patient d'accepter le taux de base rectifié.

7. Système selon la revendication 1, dans lequel le dispositif de contrôle réalise en outre l'action de sécurité en demandant à un patient de réaliser une lecture de mesure de bandelette de glycémie pour confirmer si la valeur de la glycémie mesurée est précise.

8. Système selon la revendication 1, dans lequel le dispositif de contrôle enregistre le taux de base rectifié.

9. Système selon la revendication 1, dans lequel la fourchette comprend 25 % au-dessus ou en dessous d'un taux de base préétabli.

10. Système selon la revendication 1, dans lequel une valeur absolue de l'augmentation autorisée maximale n'est pas égale à une valeur absolue de la diminution autorisée maximale.
